(19) Europäisches Patentamt
European Patent Office
Office européen des brevets

(11) **EP 4 295 763 A1**

(12) **EUROPEAN PATENT APPLICATION**

(43) Date of publication:
**27.12.2023 Bulletin 2023/52**

(21) Application number: **22193474.8**

(22) Date of filing: **01.09.2022**

(51) International Patent Classification (IPC):
*A61B 5/06* (2006.01)    *A61B 5/107* (2006.01)
*A61B 5/367* (2021.01)    *A61B 5/00* (2006.01)
*A61N 1/40* (2006.01)    *A61B 34/20* (2016.01)
*A61N 1/18* (2006.01)    *G06T 19/00* (2011.01)

(52) Cooperative Patent Classification (CPC):
**A61B 5/068; A61B 5/1076; A61B 5/367;
A61B 5/6852; A61B 5/6886; A61B 34/20;
G06T 17/20;** A61B 2505/05; G06T 2210/41

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.06.2022 US 202263354825 P**

(71) Applicant: **Koninklijke Philips N.V.
5656 AG Eindhoven (NL)**

(72) Inventors:
• **CHEN, Xiaowei
  Eindhoven (NL)**

• **LAUGHNER, Jacob
  Eindhoven (NL)**
• **WILDEBOER, Rogier Rudolf
  Eindhoven (NL)**
• **RUETTEN, Walter
  Eindhoven (NL)**
• **KAHLMAN, Josephus Arnoldus Johannes Maria
  Eindhoven (NL)**
• **IBRAGIMOV, Zalman
  Eindhoven (NL)**
• **SUDARSKY, Oded
  Eindhoven (NL)**

(74) Representative: **Philips Intellectual Property &
Standards
High Tech Campus 52
5656 AG Eindhoven (NL)**

(54) **GENERATING AN ANATOMICAL MODEL OF AN ANATOMICAL CAVITY**

(57)    A mechanism for synthesizing or generating one or more additional points that are able to contribute to a plurality of points for generating an anatomical model of an anatomical cavity. For a particular position of an interventional device within the anatomical cavity, a distance indicator responsive to a distance between the interventional device and the bounds of the anatomical cavity is determined. One or more additional points may then be generated using the distance indicator and the position of points representing the location of electrodes carried by the interventional device.

FIG. 1

EP 4 295 763 A1

**Description**

TECHNICAL FIELD

The present disclosure relates generally to the field of medical imaging, and in particular, to the generation of anatomical models using electrodes mounted on interventional devices.

BACKGROUND

Medical imaging is an important aspect in diagnosing and/or treating a subject. There is an increasing interest in the use of dielectric imaging, sometimes referred to as electroanatomical mapping, processes for imaging or modelling of an anatomical cavity and/or structure. Dielectric imaging processes facilitate high quality imaging or modelling of a subject's anatomy without the need for contrast agents and/or X-ray.

In a dielectric imaging process, two or more crossing electrical fields are induced by an array of electrodes positioned on the outside of the subject ("external electrodes"), such as on the surface of the subject. These electric fields induce position dependent electrical responses, such as a voltage response, in electrodes placed within the body (an "internal electrode"). A mapping function is used to predict or "map" the position dependent electrical response to a position in space. The position of internal electrodes can thereby be tracked by monitoring the response of the electrodes to these electric fields.

By tracking and iteratively recording the position of the internal electrodes, a model of the internal anatomy of the subject can be (re)constructed, e.g. by constructing a mesh around the recorded positions of the internal electrodes. This is because it can be generally assumed that the internal electrodes will only be located within cavities of the subject, thereby allowing the bounds of the cavity to be constructed.

One example of a suitable mechanism for constructing a model of the internal anatomy of the subject based on the response of internal electrodes to crossing electric fields induced by external electrodes is disclosed by the European Patent Application having the publication number EP 3568068 A1.

There is an ongoing desire to improve the accuracy of models constructed using the predicted positions of the internal electrodes.

SUMMARY OF THE INVENTION

The invention is defined by the claims.

There is proposed a processing system for generating an anatomical model of an anatomical cavity into which an interventional device, carrying two or more electrodes, is positioned.

The processing system comprises an input interface configured to obtain, for each of a plurality of positions of the interventional device: a set of electrical responses of each electrode carried by the interventional device to electric fields induced within the anatomical cavity; and at least one measure of an electrical property, each measure being a measure of the electrical property between a respective pair of electrodes carried by the interventional device or an electrode carried by the interventional device and a reference electrode.

The processing system further comprises a data processor configured to: for each of the plurality of positions of the interventional device: define, for each obtained set of electrical responses, a point in an Euclidean space system based on the set of electrical responses; determine, using the at least one measure of the electrical property, a distance indicator that is responsive to a distance between the interventional device and the bounds of the anatomical cavity; and when the distance indicator indicates there is more than a predetermined distance between the interventional device and the bounds of the anatomical cavity, synthesize one or more additional points in the Euclidean space system by processing the defined points in the Euclidean space system and the determined distance indicator.

The data processor is further configured to generate the anatomical model of the anatomical cavity by processing the defined points and each synthesized additional point.

The processing system optionally comprises an output interface for outputting the anatomical model or a part of the anatomical model.

The present disclosure provides a mechanism for increasing or densifying the number of points from which an anatomical model is generated. In particular, additional points are synthesized that are based on a determined distance that the interventional device lies from the bounds of the anatomical cavity.

The present disclosure relies upon a recognition that it is possible to determine or estimate a distance between an interventional device and a boundary, and that points that are synthesized to lie within this distance must represent positions lying within the anatomical cavity. This provides additional points for accurate generation of the anatomical model.

Generally, the more points that are available for generating the anatomical model, the more accurately the anatomical model represents the anatomical cavity. The advantage gained in providing additional points outweighs any disadvantage in using synthesized, simulated or synthetic points.

There is therefore proposed an approach for increasing the accuracy of the anatomical model. Proposed approaches also facilitate generation of an anatomical model at an earlier point in time than previously available as, typically, a minimum number of points are required before an anatomical model can be constructed, e.g., with a sufficiently high degree of accuracy.

The anatomical model may be a mesh. Thus, some of the points in the Euclidean space system may define a vertex or corner point of the mesh. The anatomical model may, for instance, be a polygon mesh such as a triangular mesh.

In some examples, the step of generating the anatomical model of the anatomical cavity may comprise processing only the defined points and the one or more synthesized additional points (i.e., and no other points). More particularly, the points used to generate the anatomical model may comprise only those points defined from sets of electrical responses and those synthesized using the same points defined from sets of electrical responses and the determined distance between the device and the bounds of the anatomical cavity.

The bounds of the anatomical cavity may represent tissue of a subject or patient. The anatomical cavity may represent a passage or hole within the tissue that allows for the movement of gas or liquid (e.g., the lumen of an artery or the chamber of a heart).

In some examples, the processing system is configured to output a representation of the anatomical model (or a part thereof) to a user interface. In particular, the processing system may be configured to control a user interface such as a display to provide a visual representation of the anatomical model or a part of the anatomical model. This approach provides a clinician with useful clinical information (about the anatomical cavity) to aid them in performing a medical procedure, such as a diagnosis, treatment or assessment.

The predetermined distance may, for instance, be 0 or a value larger than 0, e.g., 1mm or the like.

The electrical property may be an impedance between the respective pair of electrodes or between the electrode and the reference electrode.

As another example, the electrical property may be a difference of a first impedance and a second impedance, the first impedance being an impedance between one of the respective pair of electrodes and a reference electrode and the second impedance being an impedance between the other of the respective pair of electrodes and the reference electrode.

The impedance (and therefore electrical property) may, for instance, be a resistance, a capacitive reactance or an inductive reactance or a combination thereof.

In some examples, for each of the plurality of positions, each additional synthesized point lies no further from any of the defined points than a distance defined by the determined distance indicator.

In some examples, the data processor is configured to (e.g., when the distance indicator indicates that there is more than a predetermined distance between the bounds of the interventional device and the bounds of the anatomical cavity) synthesize one or more additional points by performing steps comprising: defining a first hypothetical line, in the Euclidean space system, that passes through at least a first pair of defined points, for the position of the interventional device, in the Euclidean space system; and synthesizing one or more additional points along the first hypothetical line, wherein each additional point is no further from any of the first pair of defined points than a first distance defined by the distance indicator.

This approach positions one or more additional points along a same trajectory or direction in which electrodes lie. When determining the distance between the interventional device and the bounds of the anatomical cavity, techniques usually determine a distance along such a first hypothetical line. This approach therefore increases the likelihood that the additional point(s) will not lie outside the bounds of the anatomical cavity, increasing the accuracy of the anatomical model.

The step of synthesizing one or more additional points along the first hypothetical line may comprise synthesizing at least one additional point to lie outside a portion of the first hypothetical line that spans between the first pair of defined points.

In this way, additional points that go beyond the extent of the interventional device can be synthesized, increasing the number of points and accuracy of the anatomical model. Of course, one or more additional points may be synthesized along the portion of the first hypothetical line that spans between the first pair of defined points.

In some examples, the data processor is configured to synthesize one or more additional points by performing steps comprising: defining a second hypothetical line, in the Euclidean space system, that passes through at least a second pair of defined points, for the position of the interventional device, in the Euclidean space system; and synthesizing one or more additional points at one or more locations, in the Euclidean space system, that are no further from a portion of the second hypothetical line that spans between the second pair of defined points than a second distance that is defined by the distance indicator.

This approach further increases the number of synthesized points, whilst ensuring that the additional points lie within an area that is not likely to include the bounds of the anatomical cavity, thereby further improving the accuracy of the anatomical model.

The step of synthesizing one or more additional points at one or more locations may comprise synthesizing at least one additional point to lie outside the portion of the second hypothetical line that spans between the second pair of defined points.

In some examples, the data processor is configured to synthesize one or more additional points by performing steps comprising: defining a zone within the Euclidean space system, the position of the zone being defined by the defined points in the Euclidean space system for the position of the interventional device and the size of the zone being defined by the distance indicator; and synthesizing one or more additional points to lie within the defined zone.

This approach defines a zone or volume in the Euclidean space system that is likely to not contain the bounds of the anatomical cavity. In this way, an envelope or boundary of where additional points should be synthesized is provided, to provide accurate densification of the points used for generating the anatomical model.

The zone preferably has a predetermined shape. The predetermined shape may, for instance, be any suitable geometric shape such as a sphere, an ellipsoid, a cylinder, a cone, a conical frustum and so on. The predetermined shape may be based on an expected shape of an electric field generated between the two or more electrodes of the interventional device.

The data processor may be configured to define, for each electrical response, a point in the Euclidean space system by performing a process comprising using a mapping function to map the obtained electrical response to a point in the Euclidean space system, wherein the mapping function is configured such that predetermined properties and/or spatial relationships of the electrodes are maintained.

The data processor may be configured to generate the anatomical model by processing the defined points and the one or more synthesized additional points to define the position of the bounds of the anatomical cavity within a 3D volume of discrete voxels representing the Euclidean space system. Thus, the anatomical model may be represented using a 3D matrix of values (defining the bounds of the anatomical cavity within a particular position/physical space), each value representing a particular voxel of the 3D volume.

There is also proposed a computer-implemented method for generating an anatomical model of an anatomical cavity into which an interventional device, carrying two or more electrodes, is positioned.

The computer-implemented method comprises obtaining, for each of a plurality of positions of the interventional device: a set of electrical responses of each electrode carried by the interventional device to electric fields induced within the anatomical cavity; and at least one measure of an electrical property, each measure being a measure of the electrical property between a respective pair of electrodes carried by the interventional device or an electrode carried by the interventional device and a reference electrode.

The computer-implemented method also comprises for each of the plurality of positions of the interventional device: defining, for each obtained set of electrical responses, a point in a Euclidean space system based on the set of electrical responses; determining, using the at least one measure of the electrical property, a distance indicator that is responsive to a distance between the interventional device and the bounds of the anatomical cavity; and when the distance indicator indicates there is more than a predetermined distance between the interventional device and the bounds of the anatomical cavity, synthesizing one or more additional points in the Euclidean space system by processing the defined points in the Euclidean space system and the determined distance indicator.

The computer-implemented method further comprises generating the anatomical model, of the anatomical cavity, by processing the defined points and each synthesized additional point.

The computer-implemented method may further comprise outputting the anatomical model or a part of the anatomical model.

The skilled person would be readily capable of adapting the computer-implemented method to perform the function of any herein described processing system, and vice versa.

There is also proposed a computer program product comprising code which, when executed by a processor circuit, causes the processor circuit to perform the steps of any herein described method. There is also proposed a non-transitory computer-readable medium or data carrier comprising or carrying the computer program product.

Any advantages of the processing system according to the present invention analogously and similarly apply to the computer-implemented method and computer program product herein disclosed.

It will be appreciated by those skilled in the art that two or more of the above-mentioned options, implementations, and/or aspects of the invention may be combined in any way deemed useful.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates a system for analyzing an anatomical cavity;
Figure 2 illustrates an approach for converting a point cloud to an anatomical model;
Figure 3 illustrates a method according to an embodiment;
Figure 4 illustrates a first approach for synthesizing one or more additional points;
Figure 5 illustrates a second approach for synthesizing one or more additional points;
Figure 6 illustrates a third approach for synthesizing one or more additional points;
Figure 7 illustrates a system and a processing system according to an embodiment; and
Figure 8 illustrates the processing system.

DETAILED DESCRIPTION OF THE EMBODIMENTS

The invention will be described with reference to the figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figures are merely schematic and are not drawn to scale. It should also be understood that the same reference numerals are used throughout the figures to indicate the same or similar parts.

Ordinal numbers (e.g. "first", "second" and so on) have been used purely to distinguish different elements from one another for the sake of clarity, and reference to a non-"first" (e.g. "second" or "third") element does not necessitate that a "first" element be present. The skilled person would be capable of relabeling any such elements as appropriate (e.g. relabeling a "second" element as a "first" element if only the second element is present).

The invention provides a mechanism for synthesizing or generating one or more additional points that are able to contribute to a plurality of points for generating an anatomical model of an anatomical cavity. For a particular position of an interventional device within the anatomical cavity, a distance indicator responsive to a distance between the interventional device and the bounds of the anatomical cavity is determined. One or more additional points may then be generated using the distance indicator and the position of points representing the location of electrodes carried by the interventional device.

Approaches are based on the realization that a distance between the interventional device and the bounds of the anatomical cavity represents a safe area to generate or synthesize additional points that would still lie within the anatomical cavity. In this way, the number of points available for generating an anatomical model can be substantially increased, thereby increasing an accuracy of the anatomical model and moving a time as which a more complete anatomical model can be generated earlier in a cavity investigation procedure.

Embodiments can be employed in any suitable clinical environment in which an anatomical cavity is being investigated.

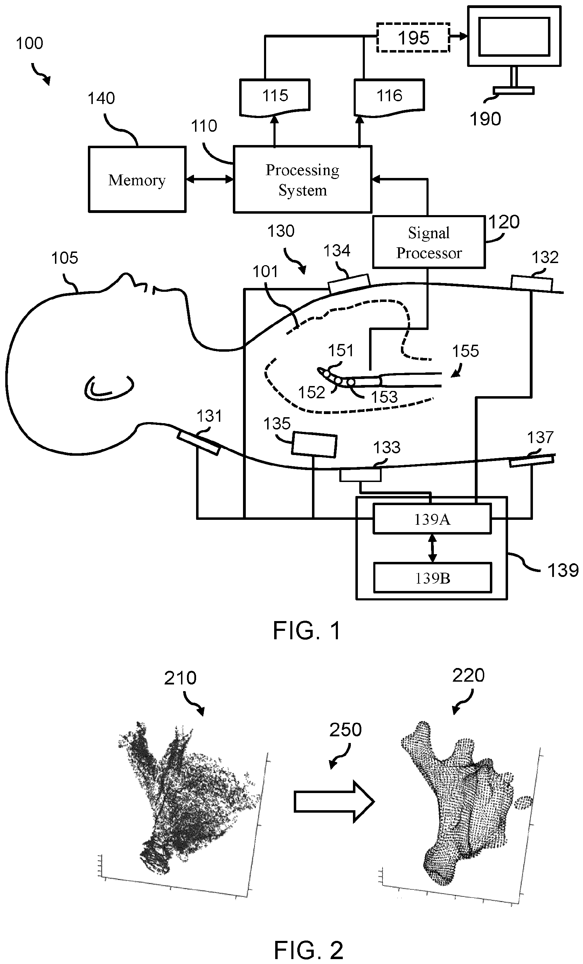

Figure 1 conceptually illustrates a system 100 for analyzing or investigating an anatomical cavity 101 of a subject 105. The anatomical cavity may, for instance, be an anatomical cavity within any suitable anatomical element of the subject, such as the heart, liver, kidney, lungs etc.

An interventional device 155 carries two or more device electrodes 151, 152, 153. The interventional device may be or comprise a catheter, although other examples will be apparent to the skilled person.

Raw signals at the device electrodes 151, 152, 153 may be filtered, sampled and/or digitized using a signal processor 120. In particular, the signal processor may (electrically) connect to the device electrodes 151, 152, 153 and process the raw signals at the field device electrodes to produce data or sets of measurements for digital processing by a processing system or other similar device. The signal processor 120 may therefore comprise appropriate circuitry for performing such functions, e.g., filtering circuitry

One approach for monitoring the location of a device electrode as the interventional device moves within the anatomical cavity is to monitor a set of electrical responses for the device electrode to crossing electric fields induced within the subject. Each electrical response in the set is an electrical response of the device electrode to a different one of the crossing electric fields. By way of example, each electrical response may be a voltage response, e.g., a detected voltage. Thus, if there are three crossing electric fields, then each set of electrical responses will comprise three different electrical responses.

As previously explained, the signal processor 120 may be appropriately configured for producing the sets of electrical responses, e.g., to sample and/or filter the raw sensor signals produced by the device electrodes for digital processing. For instance, the signal processor 120 may be able to sample and/or filter a voltage at each device electrode to produce each set of electrical responses, e.g., each set of voltage responses.

An electric field generating arrangement 130 may be configured to generate these crossing electric fields using a set of external electrodes 131, 132, 133, 134, 135, 137, which are positioned externally with respect to the subject 105. This may be performed by a field controller 139 appropriately controlling characteristics (e.g. voltage and/or current) of the electric signals provided to each external electrode.

The field controller 139 may comprise a suitable signal generator 139A for generating and transmitting signals to each field generator. The operation of the signal generator 139A may be controlled by a field controller processing system 139B of the field controller 139 (e.g., which may form part of any other processing circuitry of the system 100).

To distinguish between different electrical responses, each electric field may be generated with a different and distinguishable frequency and/or temporal code. Thus an "electrical response" may be an average or instantaneous electrical response (e.g., a voltage) at a particular frequency and/or temporal code, corresponding to the frequency and/or temporal code of the corresponding electric field.

The field controller 139 may be configured to control the electric fields to operate in the frequency range of 10-300 kHz. This frequency range is particularly useful for ensuring penetration of a subject, whilst providing a frequency range that attenuates in human tissue without significant damage/injury to the tissue.

A reference electrode 137 may serve as an electric reference for all measurements taken by electrodes, e.g. as a reference for the response of the internal electrodes.

The set of the electrical responses of a device electrode 151, 152, 153 (to the electric fields) changes as the relative position of the device electrode moves about the subject. The principle of crossing electric fields thereby facilitates tracking of the relative position of the interventional device using a processing system 110 that monitors the electrical responses of any device electrodes to the crossing electric fields.

Conceptually, each set of electrical responses can represent a location, position, point or co-ordinate in an electrical-response space or electrical-response co-ordinate system, which is an example of a Euclidean space system. If each set of electrical responses is a set of voltage responses, this can be labelled the voltage space or V-space. Such a voltage space is effectively a voltage co-ordinate system.

However, due to the inhomogeneity of the electric field(s), for clinical interpretation it is usually necessary to map the set of electrical responses of an electrode to a relative position within the subject using a suitable voltage-to-position or mapping function, sometimes called a "V2R function" - although this can be relabeled as appropriate if a different electrical response to a voltage response is obtained. The mapping function therefore converts a set of electrical responses into a location or position (e.g., set of co-ordinates) in a spatial co-ordinate system, being another example of a Euclidean space system. This mapping function will therefore determine the relative position(s) of the electrodes, and therefore the interventional device, in a spatial co-ordinate system. This spatial co-ordinate system is sometimes called an R-space. A spatial co-ordinate system is one that represents a true spatial relationship (e.g., proportionally scaled to geometric or physical distances) between elements modelled in the spatial co-ordinate system. Thus, distances in the spatial co-ordinate system are proportional to the physical distances they represent.

Generally, generating a mapping function makes use of known inter-electrode distances to calibrate for the inhomogeneity in the electric fields. Thus, it is typically necessary to know the distance between each of one or more pairs of device electrodes. Historically, these have been provided by the manufacturer or operator of the interventional device.

In this way, the mapping function is configured such that predetermined properties and/or spatial relationships of the electrodes are maintained.

Approaches for generating such a mapping function, e.g., that employ these principles, are well established in the art. Example processes are disclosed by the European Patent Applications EP 0775466 A2, EP 3568068 A1 and EP 3607879 A1.

It is possible to construct an anatomical model 115 of the anatomical cavity 101 (i.e. a cavity in which the interventional device is able to move). This process may also be performed by the processing system 110.

The anatomical model can be constructed in the electrical-response co-ordinate system or the spatial co-ordinate system, both of which are examples of a Euclidean space system. If constructed in the electrical-response co-ordinate system, it can be later mapped or transferred to the spatial co-ordinate system, e.g., using the mapping function.

A brief description of how to generate an anatomical model is provided for improved contextual understanding. This description also aids to emphasize a particular use-case scenario for data derived from a voltage-to-position function or mapping function, and therefore the clinical benefit(s) and advantage(s) of a more accurate voltage-to-position function or mapping function and a more accurate anatomical model.

Broadly, the processing system 110 may build an anatomical model 115 of an anatomical cavity 101 (e.g. a chamber, vessel or void) within a subject by processing a cloud of points. Each point represents a location or position of the interventional device or electrode in a/the Euclidean co-ordinate system (e.g., the electrical-response or spatial co-ordinate system), and could therefore be represented as a set of data, e.g. a set of co-ordinates.

More specifically, a reconstruction algorithm is used to generate an anatomical model of the (investigated part of the) anatomical cavity. The anatomical model may, for example, be a 3D mesh or surface that depicts or models the (known bounds of the) anatomical cavity. In the present disclosure, the anatomical model comprises a 3D mesh defined by a plurality of vertices, each vertex being associated with a different location in the Euclidean co-ordinate system. The 3D mesh is therefore defined in the same co-ordinate system as the determined locations of the device electrode(s). The 3D mesh may, for instance, be defined as sets of co-ordinates defining vertices of the 3D mesh.

The process of reconstructing an anatomical model from a point cloud is conceptually illustrated in Figure 2, which demonstrates a process 250 in which a cloud of points 210 ("point cloud") is transformed into an anatomical model 220. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

In particular, generating an anatomical model can comprise processing a plurality of points within a Euclidean space system to define the position of the bounds of the anatomical cavity within a 3D volume of discrete voxels representing the Euclidean space system.

Referring back to Figure 1, the anatomical model may be stored, for instance, in a memory 140 or storage unit. Of course, the anatomical model, or a part thereof, may be output for further processing and/or to a user interface 190 configured to provide a visual representation of the anatomical model.

Thus, the system 100 may comprise a user interface 190 configured to provide a visual representation of the anatomical model or a part thereof. In some examples, a display processor 195 may be configured to receive the anatomical model 115 (or a part of the anatomical model) from the processing system 110 and render the anatomical model to generate or render display data that can be received and processed by the user interface 190 for providing a visual representation of the anatomical model or a part of the anatomical model.

It is not essential that all of the anatomical model is output by the processing system 110, e.g., for rendering and/or displaying at the user interface, for storing or for further processing. Rather, only a part of the anatomical model may be output. In particular, a user may be able to select or identify a part of the anatomical model to be output (e.g., rendered and/or displayed), e.g., via a user input provided to the processing system 100 or display processor 195 via a user input interface.

Once a 3D mesh has been generated then a visual representation of the 3D mesh, or a part thereof, can be provided. Providing a visual representation of the 3D mesh or a part thereof may comprise, for example, rendering a reconstruction of the surface of the (known) anatomical cavity, or a part of the surface of the (known) anatomical cavity.

It will be apparent that once an anatomical model has been generated, it is possible to monitor the location of the device electrode(s) and/or interventional device with respect to the anatomical model. The processing system 110 may be accordingly configured to determine a location of the device electrode(s) and/or the interventional device. This may comprise identifying a new location 116 of each device electrode.

The monitored (new) location may be used, for instance, to provide a visual representation of the known bounds of the anatomical cavity (represented by the anatomical model) and the relative position of the interventional device with respect to the anatomical cavity. This provides useful clinical information to an operator of the interventional device, e.g., to track the progress of the interventional device and/or view the shape/structure/size of the anatomical cavity in which the interventional device is to be moved or is moving.

The present invention proposes a technique for increasing the number of points or positions used for generating the anatomical model. This process can be labelled densification, and improves the accuracy of the anatomical model. Generally, the more points that are available for generating the anatomical model, the greater the accuracy of the anatomical model.

The proposed technique synthesizes additional points based on a determined distance between the interventional device and the bounds of the anatomical cavity when the electrical response corresponding to a particular set of points was captured or sampled.

This technique is carried out by the processing system 110, which can be alternatively labelled a mapping system.

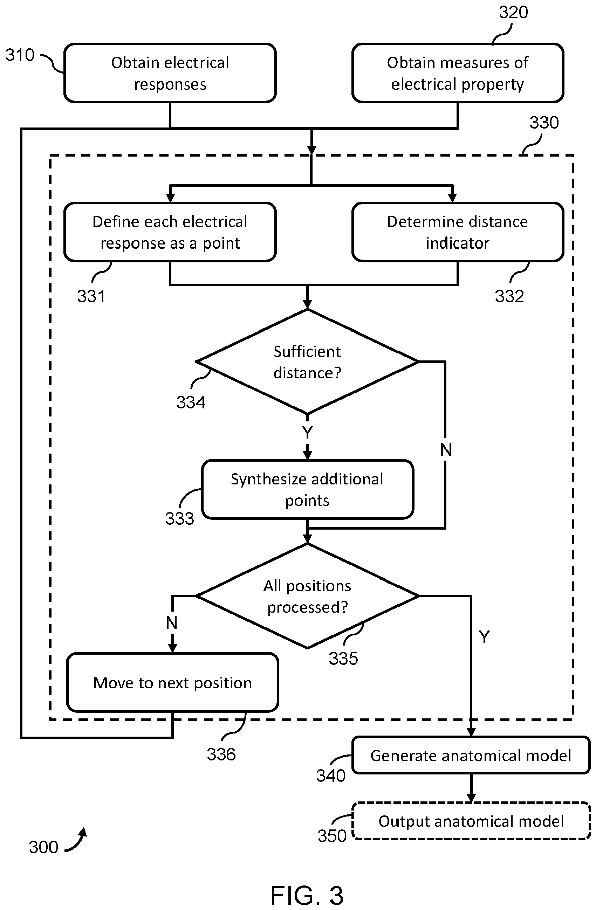

Figure 3 is a flowchart illustrating a computer-implemented method 300 according to the herein proposed technique. The computer-implemented method 300 may be implemented or performed by the processing system 110.

The method 300 is for generating an anatomical model of an anatomical cavity into which an interventional device, carrying two or more electrodes, is positioned. The method 300 may be a passive method that is carried out whilst the interventional device is within the anatomical cavity of the subject. None of the steps performed by the method require any direct physical interaction between the device and the subject, but rather relate to approaches for the mere processing of data.

The method 300 comprises a step 310 of obtaining or receiving, for each of a plurality of positions of the interventional device, a set of electrical responses of each electrode carried by the interventional device to electric fields induced within the anatomical cavity.

It has previously been explained how the set of electrical responses changes dependent upon the position of the interventional device with respect to the (crossing) electric fields. Thus, each set of electrical responses is effectively a measurement of the crossing electric fields.

The method 300 also comprises a step 320 of obtaining or receiving, for each of the (same) plurality of positions of the interventional device, at least one measure of an electrical property, each measure being a measure of the electrical property between a respective pair of electrodes carried by the interventional device or between an electrode carried by the interventional device and a reference electrode.

The measure of the electrical property should be a measure that can be processed in order to estimate or determine a distance between the interventional device and the bounds of the anatomical cavity, e.g., anatomical tissue.

One suitable example is an impedance between the pair of device electrodes. Approaches for determining an impedance between two device electrodes are established in the art.

As one example, an impedance can be determined by injecting a current at one device electrode, and measuring or sampling a voltage at each of the pair of device electrodes responsive to the injected current (e.g., at the specific frequency of the injected current). The injected current may be a current injected towards a/the reference electrode. Thus, an impedance $Z_{J,K}$ between device electrodes J and K (forming a device electrode pair) can be determined using the following equation:

$$Z_{J,K} = \frac{V_{J,L} - V_{K,L}}{I_L} \qquad (1)$$

where $I_L$ is a current injected at electrode L, $V_{J,L}$ is a voltage at electrode J responsive to the current $I_L$, and $V_{K,L}$ is a voltage at electrode K responsive to the current $I_L$. The electrode L may be one of the device electrodes J, K, or a different device electrode.

Another suitable example is a difference in impedances between each electrode and a reference electrode. This difference is therefore a difference between a first impedance and a second impedance, the first impedance being an impedance between one of the respective pair of electrodes and a reference electrode and the second impedance being an impedance between the other of the respective pair of electrodes and the reference electrode.

Thus, a difference $D_{J,K}$ for a device electrode pair J, K can be calculated using the following equation:

$$D_{J,K} = \frac{V_{J,L1} - V_{R,L1}}{I_{L1}} - \frac{V_{K,L2} - V_{R,L2}}{I_{L2}} \qquad (2)$$

where $I_{L1}$ is a current injected at electrode L1, $I_{L2}$ is a current injected at electrode L2, $V_{J,L1}$ is a voltage at electrode J responsive to the current $I_{L1}$, $V_{K,L2}$ is a voltage at electrode K responsive to the current $I_{L2}$, $V_{R,L1}$ is a voltage at the reference electrode responsive to the current $I_{L1}$, and $V_{R,L2}$ is a voltage at the reference electrode responsive to the current $I_{L2}$. The electrode L1 is preferably electrode J, and the electrode L2 is preferably electrode K, for improved accuracy and relevance of the determined difference $D_{J,K}$. However, it is possible to for other electrodes carried by the interventional device to act as the electrode L1, L2.

Yet other suitable examples of suitable electrical properties include: a resistance, a capacitive reactance and/or an inductive reactance or a combination thereof between the two electrodes. These properties represent some other measurable electrical properties that change with distance between the interventional device and the bounds of the anatomical cavity.

Yet other suitable examples include an impedance, resistance, capacitive reactance and/or an inductive reactance or a combination thereof between an electrode and a reference electrode.

Any used measure of the electrical property may be gated (e.g., selectively sampled) or corrected for cardiac motion in order to minimize the effect of cardiac contraction on the distance reading. Gating a measure means to consistently sample the measure at times such that the effect of cardiac contraction on consecutive measurements is minimal or negligible.

It is also recognized that measures of an electrical property (such as impedance values) are affected by at least an electrode surface area and distance between them. A calibration process can be used to mitigate or eliminate this effect. One approach is to divide the measured/sampled measures of an electrical property by a reference value. The reference value can be determined during a "zeroing" procedure, which can be initiated when enough volume has been imaged for the blood pool (or center of the cavity) to be distinguishable. In particular, the 5th percentile value (for the measure of the electrical property) during a zeroing process can be used as the reference value. As another example, the reference value can be (e.g., at least temporarily or until a zeroing procedure is performed) defined with a pre-set value.

The above examples of determining the measure of the electrical property are provided for improved contextual understanding. Although some embodiments of a processing system that carries out method 300 may perform this process of actively determining the measure of the electrical property, this is not essential for carrying out method 300 itself. Rather, step 320 merely comprises a passive process of obtaining or receiving the measure(s) of the electrical property for each of the plurality of positions of the interventional device, e.g., from a memory, storage device or other processing system.

The method 300 then performs a synthesis process 330. The synthesis process 330 is configured to synthesize or create additional points within a Euclidean space system, beyond those defined directly by the obtained or received sets of electrical responses (obtained in step 310).

The synthesis process is performed for a plurality of positions of the interventional device, i.e., for each set of electrical responses obtained in step 310.

The synthesis process 330 comprises a step 331 of defining, for each set of responses, a point (or position) in a Euclidean space system based on the set of electrical responses. This effectively determines a position of each device electrode within the Euclidean space system for a single location/position of the interventional device. The Euclidean space system may be the electrical-response co-ordinate system or the spatial co-ordinate system, previously described.

Thus, step 331 may simply comprise defining, for each device electrode, a point in an electrical-response co-ordinate system based on each electrical response in the set of electrical responses for that device electrode. For instance, the value of each electrical responses may represent a position along a respective axis, to thereby effectively identify a set of co-ordinates in the electrical-response co-ordinate system for each device electrode. This approach defines points within the electrical-response co-ordinate system.

Another approach may process each set of electrical responses using the mapping function, to determine a point, position or location within the spatial co-ordinate system. Thus, a position of each device electrode within the spatial co-ordinate system can be identified. This approach defines points within the spatial co-ordinate system.

Thus, step 331 may comprise defining, for each electrical response, a point in the Euclidean space system by performing a process comprising using a mapping function to map the obtained electrical response to a point in the Euclidean space system, wherein the mapping function is configured such that predetermined properties and/or spatial relationships of the electrodes are maintained.

More generally, step 331 defines each electrical response in the set as a point, e.g., a set of co-ordinates, in a Euclidean space system.

The synthesis process 330 also comprises a step 332 of determining, using the at least one measure of the electrical property, a distance indicator that is responsive to a distance between the interventional device and the bounds of the anatomical cavity. The distance between the interventional device and the bounds of the anatomical cavity can be labelled a "device-bounds distance".

The determined distance indicator may be expressed as a binary value (e.g., "0" representing near and "1" representing far), a categorical value (e.g., "Very Close", "Near" and "Far") or a numeric value, representing a predicted distance between the interventional device and the bounds of the anatomical cavity.

A binary or categorical value for a distance indicator may indicate a predicted range of distances for the distance between the interventional device and the bounds of the anatomical cavity. For instance, a binary value may indicate a prediction of whether or not the distance between the interventional device and the bounds of the anatomical cavity is above or below a predetermined (non-zero) distance threshold. A categorical value may indicate a prediction of into which of a plurality of predetermined ranges the device-bounds distance (between the interventional device and the bounds of the anatomical cavity) falls.

In this way, each binary or categorical value is associated with a corresponding range of (predicted) distances between the interventional device and the bounds of the anatomical cavity.

It is recognized that the measure of the electrical property between two electrodes is responsive or changes responsive to proximity to tissue or the bounds of the anatomical cavity. Various approaches for processing the measure(s) to determine or predict the distance indicator could be used.

In one example, the step 332 converts from the measure(s) to a distance indicator (e.g., a predicted distance) using an analytical calculation derived from first principles.

In another example, the step 332 converts from the measure(s) to the distance indicator (e.g., a predicted distance) using an equation or a lookup table derived from electric field simulations or measurements.

In another example, the step 332 converts from the measure(s) the distance indicator (e.g., a predicted distance) using a machine-learning algorithm (e.g., a neural network) to process the estimated distances.

A machine-learning algorithm is any self-training algorithm that processes input data in order to produce or predict output data. Here, the input data comprises one or more measures of an electrical property and the output data comprises a distance to the bounds of the anatomical cavity.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. The training input data entries correspond to example measures of the electrical property. The training output data entries correspond to a distance indicator that indicates or is responsive to a distance to the bounds of the anatomical cavity. The training dataset is used to iteratively train the machine-learning algorithm.

An initialized machine-learning algorithm is applied to each training input data entry to generate predicted output data entries, which here each indicate a prediction of the distance indicator. An error between the predicted output data entries and the corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

An example process for generating a suitable training dataset is hereafter described. In particular, a process for generating a training input data entry and a corresponding training output data entry is described.

Firstly, an anatomical model of an anatomical cavity can be obtained. An interventional device carrying two or more electrodes may be positioned at a particular position within the same anatomical cavity. To obtain a training input data entry, a set of electrical responses of at least one of the two or more electrodes is used to determine the position of interventional device with respect to anatomical model. The distance between this determined position and the bounds of the anatomical cavity (defined by the anatomical model) is then determined, to define the training output data entry, e.g. by setting the value of the training input data entry to be equal to the determined distance or setting a binary/categorical value for the distance indicator using the determined distance (e.g., comparing to predetermined ranges or threshold values). The training input data entry is defined using the measure of electrical property at the position of the interventional device.

The above process can be repeated for different positions of the interventional device to generate additional data for the training dataset. Moreover, the above process could be repeated for different subjects to improve the generality of the machine-learning algorithm.

With continued reference to Figure 3, the step 332 may comprise comparing the measure(s) to one or more predetermined ranges. Each range may be associated with a different value for the distance indicator. For instance, if the distance indicator provides a categorical value, different ranges of the measure(s) can be associated with a different category (e.g., "very close", "close" or "far"). If the distance indicator provides a binary value, falling within a first range may cause the distance indicator to have a first binary value indicating the interventional device is near the bounds of the anatomical cavity, otherwise (i.e., when the measure of the electrical property falls outside the first range) the distance indicator has a second binary value indicating the interventional device is far from the bounds of the anatomical cavity.

By way of example, there may be an upper value and a lower value for the measure(s). A measure falling below the lower value may indicate the device is "far" from the bounds of the anatomical cavity. A measure falling between the lower and upper values may indicate the device is "close" to the bounds of the anatomical cavity. A measure falling above an upper value may indicate the device is "very close" to the bounds of the anatomical cavity.

This approach recognizes that impedance will increase with increased proximity to the bounds of the anatomical cavity.

The values for the bounds of the predetermined ranges (e.g., the upper and lower values) may be predetermined.

Alternatively, the values for the bounds of the predetermined ranges the upper and lower values may be calibrated during a calibration or zeroing procedure, e.g., performed by the operator of the interventional device.

For instance, during a calibration procedure a measure of the electrical property ("calibration measure") can be taken when the interventional device is known to be at least a predetermined distance away from the bounds of the anatomical cavity, e.g., in a blood pool. The bounds of the ranges may be set or defined using the determined measure of the electrical property. For instance, the bounds of the range(s) may be set to be equal to a predetermined percentage of this determined measure. For instance, an "upper value" may be set to be equal to 150% of the calibration measure and the lower value may be set to be equal to 120% of the calibration measure. Other suitable predetermined percentages will be apparent to the skilled person.

In some examples, the process used in step 332 to convert from a measure of an electrical property to distance to the anatomical bounds can be updated or refined during an imaging procedure, e.g., as the anatomical model is iteratively updated.

In particular, it is possible to monitor the electrical property to detect contact or no-contact between the interventional device and the anatomical bounds. There will be a sharp or sudden change in the measure of the electrical property when contact is made (compared to when no-contact is made). This can be detected and used to identify positions ("known bound positions") in the Euclidean space known to be on the bounds of the anatomical cavity. Approach for detecting contact between an interventional device and the bounds of the anatomical cavity are known in the art, as described by WO 2019/215721 A1 and/or US 2012/283715 A1,

It is possible to determine a distance between the interventional device and the known bound positions, e.g., using a relative position of the interventional device in the spatial co-ordinate position and the known bound position(s) in the spatial co-ordinate system. This information can be used to revise or update the process used in step 332 to convert from a measure of an electrical property to a measure of a distance to the anatomical bounds, e.g., update the values for the bounds of the one or more predetermined ranges.

The process 330 then moves to a step 333 of synthesizing one or more additional points in the Euclidean space system. This is achieved by processing the defined points in the Euclidean space system (defined in step 331) and the determined distance between the interventional device and the bounds of the anatomical cavity (determined in step 332).

In some examples, step 333 is performed only when the distance indicator indicates that there is a non-zero distance between the interventional device and the bounds of the anatomical cavity.

In some examples, step 333 is performed only when the distance indicator indicates that the distance between the interventional device and the bounds of the anatomical cavity is no less than a predetermined distance.

Thus, step 333 may not be performed if the distance indicator indicates that the interventional device is touching the bounds of the anatomical cavity or is within a certain predetermined distance of the anatomical cavity (e.g., so close as to be almost touching).

This may be determined in a determination step 334. Determination step 334 may comprise processing the distance indicator to determine whether or not the distance between the interventional device and the bounds of the anatomical cavity is more than a predetermined distance. If a positive determination is made, the process 330 may move to step 333, otherwise, step 333 may be skipped.

In some examples, the one or more additional synthesized points may lie no further from any of the defined points than a distance defined by the determined distance indicator. This distance defined by the distance indicator may be equal to, or a predetermined fraction or percentage (e.g., 75% or 90%), of the value of a lower bound of a range associated with the determined distance indicator (for binary/categorical distance indicators). As another example, the distance may be equal to, or a predetermined fraction or percentage (e.g., 75% or 90%), the value of the determined distance indicator (for numeric distance indicators).

Step 333 effectively creates points representing virtual electrodes. These virtual electrodes may include virtual electrode along the (axis of the) interventional device, e.g., between these real electrodes by performing interpolation. The virtual electrodes may also or otherwise include virtual electrodes that are added in front of the tip electrode (i.e., the most distally positioned real electrode) by performing extrapolation. Virtual electrodes may also or otherwise be added around all other electrodes (real, virtual) in any plane perpendicular to the axis of the interventional device.

These points created in step 333 are then effectively added to existing or defined points to form a point cloud, thereby densifying the effective point cloud previously available.

To improve processing efficiency, the distance between any pair of the additional point(s) and the defined points may be no less than a predetermined minimum distance. This may reduce the chance of an extremely large number of additional points being generated.

The predetermined minimum distance may be a distance corresponding to a voxel size of an intended rendering of an anatomical model produced using the defined and additional points. In particular, the predetermined minimum distance may be equal to the size of the smallest dimension of a voxel for an intended rendering of an anatomical model produced using the defined and additional points. For instance, if a voxel of an intended rendering has a size of $1mm^3$, then the predetermined minimum distance may be 1mm. As another example, the predetermined minimum distance may be a fixed distance, e.g., 1mm or 0.5mm.

In another example, the predetermined minimum distance may a distance defined by a user input, e.g., received at a user input interface.

More specific approaches for performing step 333 are described later in this disclosure.

As previously explained, the synthesis process is performed for all the positions of the interventional device. Determination of whether all positions have been processed may be made in a determination step 335. In response to a positive determination in step 335, the synthesis process 330 is ended. Otherwise, the synthesis process moves to the next position in a step 336, and starts from the beginning.

After the synthesis process 330 is completed, the method 300 moves to step 340 of generating an anatomical model of the anatomical cavity. Step 340 processes the defined points and the one or more synthesized additional points to generate the anatomical model.

Approaches for generating an anatomical model using a plurality or cloud of points have been previously described, and can be employed for use in step 340, such as those discussed in Berger, Matthew, et al. "A survey of surface reconstruction from point clouds." Computer Graphics Forum. Vol. 26. No. 1. 2017.

The anatomical model may be constructed in the electrical-response co-ordinate system or the spatial co-ordinate system. Thus, where appropriate, step 340 may comprise mapping the defined points and one or more synthesized additional points to the spatial co-ordinate system from the electrical-response co-ordinate system.

Optionally, the method 300 further comprises a step 350 of outputting the anatomical model (i.e., all or an entirety of the anatomical model) or a part of the anatomical model.

Step 350 may comprise providing a visual representation of the anatomical model, or a part thereof, at a user interface, e.g., a screen or display. This provides a clinician or operator with a view of the anatomical cavity for aiding in the performance of a clinical procedure or analyzing the anatomical cavity.

Step 350 may comprise storing the anatomical model in a storage unit or memory, e.g., for later retrieval and/or analysis.

Step 350 may comprise providing the anatomical model to a further processing system for further processing, e.g., automated analysis, filtering, supplementing with additional information and so on.

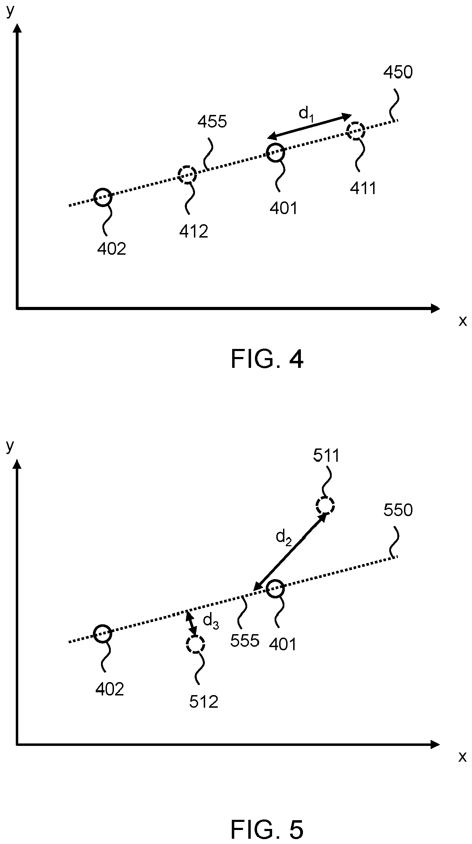

Figures 4 to 6 conceptually illustrate various approaches for synthesizing one or more additional points in a Euclidean space system by processing the defined points in the Euclidean space system and the determined distance between the interventional device and the bounds of the anatomical cavity for a particular position of the interventional device.

In Figures 4 to 6, only two dimensions x, y of a Euclidean space system are illustrated, for clarity and ease of explanation. However, it will be evident that a Euclidean space system will typically comprise three dimensions, e.g., x, y, z, representing a three-dimensional space.

Figures 4 to 6 all illustrate a first point 401 and a second point 402 in the Euclidean space system, each point being a point defined using a set of electrical responses of a device electrode. Each point represents a different device electrode of the interventional device. The first and second points thereby form a pair of defined points.

Figure 4 illustrates a first approach to synthesizing one or more additional points. In this approach, to synthesize one or more additional points, a first hypothetical line 450 is defined. The first hypothetical line 450 passes through at least one pair of defined points, here: the first point 401 and the second point 402.

One or more additional points 411, 412 are then synthesized along the first hypothetical line 450. The additional points are positioned so that a distance di between each additional point 411, 412 and any one of the defined points 401, 402 is less than a first distance defined by the distance indicator.

The value of the first distance is responsive to the distance indicator, and may depend upon the data type of the distance indicator. Where the distance indicator is a numeric indicator of distance, the first distance may be equal to the value of the distance indicator or a predetermined fraction or percentage (e.g., 75% or 90%) of the value of the distance indicator. Where the distance indicator is a binary or categorical value (thereby indicating a range into which the device-bounds distance falls), the first distance may be equal to the value of the distance indicator or a predetermined fraction or percentage (e.g., 75% or 90%) of the lower bound of the range associated with the value of the distance indicator.

This approach effectively places at least one additional point along the same trajectory as the defined points 401, 402.

By restricting the additional point(s) to not lie further than the determined distance, a likelihood that the additional point will lie at a position occupied by the bounds of the anatomical cavity is significantly reduced. This significantly increases an accuracy of any anatomical model generated using the additional point(s).

Preferably, at least one of the additional points 411, such a first additional point 411, is located outside a portion 455 of the hypothetical line that spans between the pair of defined points 401, 402. This effectively expands the area or space occupied by points or synthesized points, increasing the accuracy of any anatomical model generated using such points.

This approach is particularly advantageous when one of the pair of defined points represents a tip electrode, e.g., an electrode positioned most distally to the interventional device.

In preferable examples, one of the pair of defined points (defining the hypothetical first line) is a point representing the most distally positioned device electrode of the interventional device. In such examples, at least one additional point may be synthesized to lie upon the first hypothetical line 450 such that the defined point representing the most distally positioned device electrode lies between the at least one additional point and the other defined point in the pair of defined points.

Thus, if the first point 401 is a point representing the most distally located device electrode, then the first additional point 411 fulfills this restriction.

Of course, in some examples at least one additional point, such as a second additional point 412, is located between the pair of defined points. Thus, one or more additional points can be interpolated between known or defined points.

Preferably, as many additional points as possible may be generated that lie on the first hypothetical line that: i) do not lie further than the first distance from any of the defined points and, ii) are located such that the distance between any pair of the additional point(s) and the defined points is no less than a predetermined minimum distance. This maximizes the number of points that are generated, whilst ensuring processing efficiency. Examples of suitable predetermined minimum distances have been previously described.

More than one first hypothetical line, e.g., connecting different pairs of electrodes, can be generated for synthesizing different additional points. The illustrated example demonstrates a single hypothetical line for the purposes of clarity.

The first hypothetical line may be a straight line. In another example, the first hypothetical line may be a curve or higher order polynomial.

The precise shape of the first hypothetical line may depend upon the type and/or characteristics of the interventional device. For instance, if the interventional device is a rigid, linear structure, then the first hypothetical line may be a straight line. If the interventional device is a flexible structure, then the first hypothetical line may be curve.

Figure 5 illustrates a second approach to generating one or more additional points. In this approach, to synthesize one or more additional points, a second hypothetical line 550 is defined. The second hypothetical line 550 passes through at least one pair of defined points, here: the first point 401 and the second point 402.

One or more additional points 511, 512 are then synthesized at a respective one or more locations, in the Euclidean space system, that are no further from a portion 555 of the second hypothetical line 555 that spans between the second pair of defined points than a second distance that is defined by the distance indicator.

Thus, a distance $d_2$, $d_3$ between each synthesized additional point and the portion of the second hypothetical line spanning between the pair of defined points 401, 402 is less than the second distance.

The value of the second distance is responsive to the distance indicator, and may depend upon the data type of the distance indicator. Where the distance indicator is a numeric indicator of distance, the second distance may be equal to the value of the distance indicator or a predetermined fraction or percentage (e.g., 75% or 90%) of the value of the distance indicator. Where the distance indicator is a binary or categorical value (thereby indicating a range into which the device-bounds distance falls), the second distance may be equal to the value of the distance indicator or a predetermined fraction or percentage (e.g., 75% or 90%) of the lower bound of the range associated with the value of the distance indicator.

In some examples, the value of the second distance may change with position along the second hypothetical axis. In particular, if one of the pair of defined points (defining the second hypothetical line) is a point representing the most distally positioned device electrode of the interventional device, the value of the second distance may increase responsive to proximity to the defined point representing the most distally positioned device electrode. The maximum value for the second distance may be the value of the distance indicator or lower bound of the associated range (or the predetermined fraction/percentage thereof). This approach recognizes that a device-bounds distance calculation is more accurate closer to the more distally positioned electrode, thereby improving a likelihood that the additional points will fall within areas representing the anatomical cavity, increasing an accuracy of any subsequently generated anatomical model.

This approach effectively places at least one additional point to surround a known position of the interventional device. This approach appreciates that the space between electrodes of an interventional will be at least the determined device-bounds distance from the bounds of the anatomical cavity, such that points can be positioned at locations no further from this space to accurately densify the points available for generating an anatomical model.

Preferably, at least one additional point lies outside the portion of the second hypothetical line that spans between the second pair of defined points. Thus, at least one of the additional points may not be located on the portion of the second hypothetical line.

In preferable examples, one of the pair of defined points (defining the second hypothetical line) is a point representing the most distally positioned device electrode of the interventional device. Preferably, at least one additional point 511 is synthesized to lie at a location at which:

a distance between the additional point and the defined point 401 representing the most distally positioned device electrode of the interventional device is less than a distance between the additional point and the other 402 of the pair of defined points; and
a distance between the additional point and the other 402 of the pair of defined points is greater than a distance between the defined point 401 representing the most distally positioned device electrode of the interventional device and the other 402 of the pair of defined points

This approach will synthesize at least one additional point in front of the tip electrode, i.e., performing extrapolation. This is particularly advantageous as it synthesizes points representing areas or locations that have not yet been reached by the interventional device, improving the accuracy of a subsequently generated anatomical model.

Preferably, as many additional points as possible may be generated that: i) do not lie further than the second distance from the second hypothetical axis and, ii) are located such that the distance between any pair of the additional point(s) and the defined points is no less than a predetermined minimum distance. This maximizes the number of points that are generated, whilst ensuring processing efficiency. Examples of suitable predetermined minimum distances have been previously described.

The second hypothetical line may be a straight line. In another example, the second hypothetical line may be a curve or higher order polynomial.

The precise shape of the second hypothetical line may depend upon the type and/or characteristics of the interventional device. For instance, if the interventional device is a rigid, linear structure, then the second hypothetical line may be a straight line. If the interventional device is a flexible structure, then the second hypothetical line may be curve.

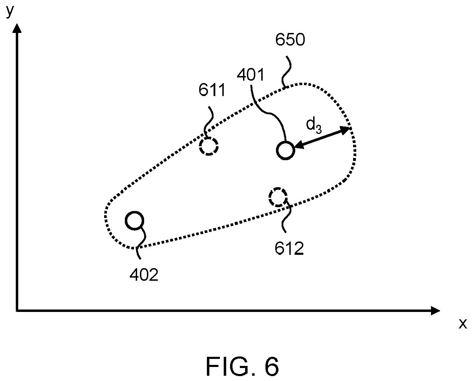

Figure 6 illustrates a third approach to generating one or more additional points. In this approach, to synthesize one or more additional points, a zone 650 is defined within the Euclidean space system. A zone is a region or volume surrounding the one or more additional points.

The position of the zone is defined by the location or position of the defined points 401, 402 in the Euclidean system. Thus, as the location or position of the defined points changes, so would the position of the zone 650. In particular, the zone is positioned to encompass or surround the defined points 401, 402.

The size of the zone is defined by the distance indicator. In particular, the larger the device-bounds distance (as indicated by the distance indicator), the larger the size of the zone. Similarly, the smaller the device-bounds distance (as indicated by the distance indicator), the smaller the size of the zone.

In particular examples, the size of the zone may be defined such that the most distant part of the zone from any of the defined points is no more than a distance represented or defined by the distance indicator or a lower bound of the range represented by the distance indicator.

For improved safety, in some examples, the size of the zone may be defined such that the most distant part of the zone from any of the defined points is no more than a distance defined by the distance indicator. This distance defined by the distance indicator may be equal to, or a predetermined fraction or percentage (e.g., 75% or 90%), of the value of a lower bound of a range associated with the determined distance indicator (for binary/categorical distance indicators). As another example, the distance may be equal to, or a predetermined fraction or percentage (e.g., 75% or 90%), the value of the determined distance indicator (for numeric distance indicators).

One or more additional points are then synthesized within the defined zone. The synthesized points may be evenly distributed throughout the defined zone. In some examples, one or more additional points are synthesized along the boundary of the defined zone. In the context of the present disclosure, a point on the boundary of the defined zone is considered to be within the defined zone.

The shape of the zone may be predetermined.

The shape can be any suitable geometric shape, such as a sphere, an ellipsoid, a cylinder, a cone, a conical frustum and so on. These provide suitable examples of shapes that can define a region in which one or more additional points are synthesized.

In one examples, the shape may represent an expected shape of an electric field generated between the two or more electrodes of the interventional device. The expected shape may, for instance, be an ellipsoidal shape. It is recognized that the measure of an electrical property will depend upon the position relative to the electric field between the two or more electrodes (e.g., the distance from the bounds of the anatomical cavity to the electric field will change the measure of the electrical property). By setting the expected shape to follow the expected electric field, a particularly accurate zone for the synthesized points can be defined.

This effect is particularly enhanced when the pair of defined points represent a pair of electrodes that provide an electrical measure for generating the distance indicator (e.g., in step 332).

Preferably, as many additional points as possible may be generated that: i) lie within the zone 650 and, ii) are located such that the distance between any pair of the additional point(s) and the defined points is no less than a predetermined minimum distance. This maximizes the number of points that are generated, whilst ensuring processing efficiency. Examples of suitable predetermined minimum distances have been previously described.

In all described embodiments, at least one additional point is synthesized for each of a plurality of positions of the interventional device for which the interventional device is not touching and/or is no closer than a predetermined distance to the bounds of the anatomical cavity. The at least one additional point preferably comprises at least two additional points, e.g., at least 10 additional points, e.g., at least 20 additional points.

The greater the number of overall points for generating the anatomical model, the greater the accuracy of the anatomical model.

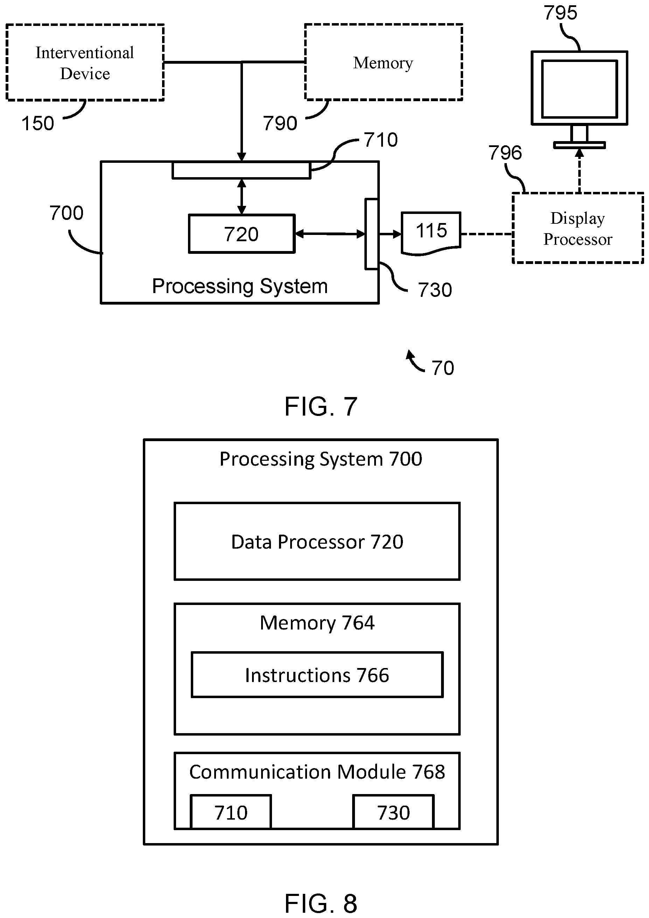

Figure 7 is a block diagram illustrating a processing system 700 according to an embodiment. The processing system 700 is configured to carry out a method according to an embodiment, such as the method 300 described with reference to Figure 3.

The processing system 700 may form part of a system 70, which is itself an embodiment of the invention.

In particular, the processing system comprises an input interface 710.

The input interface is configured to obtain, for each of a plurality of positions of the interventional device: a voltage response of each electrode carried by the interventional device to electric fields induced within the anatomical cavity; and at least one measure of an electrical property, each measure being a measure of the electrical property between a respective pair of electrodes carried by the interventional device.

Thus, the input interface 710 may carry out steps 310 and 330 of method 300. In other words, steps 310 and 330 may be performed using an input interface.

The input interface 710 may obtain this data from the interventional device 150 or from a memory 790, e.g., storing data previously generated by the interventional device.

The input interface 710 may comprise any electronic circuitry and/or logic circuitry to facilitate direct or indirect communication of data from one or more external device to the processing system 700.

The processing system also comprises a data processor 720.

The data processor 720 is configured to: for each of the plurality of positions of the interventional device: define, for each obtained voltage response, a point in a Euclidean space system based on the voltage response; determine, using the at least one measure of the electrical property, a distance between the interventional device and the bounds of the anatomical cavity for each of the plurality of positions; synthesize one or more additional points in the Euclidean space system by processing the defined points in the Euclidean space system and the determined distance between the interventional device and the bounds of the anatomical cavity.

The data processor 720 is further configured to generate an anatomical model, of the anatomical cavity, by processing the defined points and the one or more synthesized additional points.

Thus, the data processor 720 may carry out process 330 and step 340 of method 300. In other words, process 330 and step 340 may be performed using a data processor.

The processing system 700 optionally comprises an output interface 730 for outputting the anatomical model or a part of the anatomical model. The output interface 730 may output the anatomical model, or a part thereof, to a further processing system and/or a display unit or user interface 795.

Thus, the system 70 may comprise a user interface 795 configured to provide a visual representation of the anatomical model.

In some examples, the system 70 comprises a display processor 796 configured to receive the anatomical model 115 from the processing system 700 and render the anatomical model to generate display data that can be received and processed by the user interface 190 for providing a visual representation of the anatomical model.

Although illustrated as a separate element, the display processor 796 may be an aspect or part of the processing system 700.

Thus, the output interface 730 may perform step 350 of method 300. In other words, steps 350 may be performed using an output interface.

The input interface 710 may comprise any electronic circuitry and/or logic circuitry to facilitate direct or indirect communication of data from the processing system 700 to one or more one or more external device.

The skilled person would be readily capable of adapting the processing system to carry out the steps of any herein described computer-implemented method, and vice versa.

Figure 8 is a schematic diagram of a processing system according to an embodiment, e.g., the processing system 700 previously described with reference to Figure 7.

As shown, the processing system 700 may include data processor 720, a memory 764, and a communication module 768. These elements may be in direct or indirect communication with each other, for example via one or more buses.

The data processor 720 may include a central data processor (CPU), a digital signal processor (DSP), an ASIC, a controller, an FPGA, another hardware device, a firmware device, or any combination thereof configured to perform the operations described herein. The data processor 720 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 764 may include a cache memory (e.g., a cache memory of the data processor 720), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 764 includes a non-transitory computer-readable medium. The non-transitory computer-readable medium may store instructions. For example, the memory 764, or non-transitory computer-readable medium may have program code recorded thereon, the program code including instructions for causing the processing system 700, or one or more components of the processing system 700, particularly the data processor 720, to perform the operations described herein. For example, the processing system 700 can execute operations of the method 300.

Instructions 766 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 764, with the code recorded thereon, may be referred to as a computer program product.

The communication module 768 can include any electronic circuitry and/or logic circuitry to facilitate direct or indirect communication of data between the processing system 700 and any external devices. In that regard, the communication module 768 comprises the input interface 710 and the output interface 720 for the processing system 700. In some instances, the communication module 768 facilitates direct or indirect communication between various elements of the processing system 700, e.g., using a bus or the like.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

It will be appreciated that different disclosed processing systems may form part of a single processing system, e.g., each disclosed processing system is a sub-system of a single processing system. In the context of the present disclosure, this means that a single system could perform the actions of a processing system 110 and/or a field controller processing system 139B as herein described.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A processing system for generating an anatomical model of an anatomical cavity into which an interventional device, carrying two or more electrodes, is positioned, the processing system comprising:

   an input interface configured to obtain, for each of a plurality of positions of the interventional device:

      a set of electrical responses of each electrode carried by the interventional device to electric fields induced within the anatomical cavity; and
      at least one measure of an electrical property, each measure being a measure of the electrical property between a respective pair of electrodes carried by the interventional device or an electrode carried by the interventional device and a reference electrode ;

   a data processor configured to:

for each of the plurality of positions of the interventional device:

define, for each obtained set of electrical responses, a point in a Euclidean space system based on the set of electrical responses;
determine, using the at least one measure of the electrical property, a distance indicator that is responsive to a distance between the interventional device and the bounds of the anatomical cavity; and
when the distance indicator indicates there is more than a predetermined distance between the interventional device and the bounds of the anatomical cavity, synthesize one or more additional points in the Euclidean space system by processing the defined points in the Euclidean space system and the determined distance indicator, and

generate the anatomical model, of the anatomical cavity, by processing the defined points and each synthesized additional point, and

optionally, an output interface for outputting the anatomical model or a part of the anatomical model.

2. The processing system of claim 1, wherein the electrical property is either:

an impedance between the respective pair of electrodes; or
a difference of a first impedance and a second impedance, the first impedance being an impedance between one of the respective pair of electrodes and a reference electrode and the second impedance being an impedance between the other of the respective pair of electrodes and the reference electrode.

3. The processing system of any of claims 1 to 2, wherein, for each of the plurality of positions, each additional synthesized point lies no further from any of the defined points than a distance defined by the determined distance indicator.

4. The processing system of any of claims 1 to 3, wherein the data processor is configured to synthesize one or more additional points by performing steps comprising:

defining a first hypothetical line, in the Euclidean space system, that passes through at least a first pair of defined points, for the position of the interventional device, in the Euclidean space system; and
synthesizing one or more additional points along the first hypothetical line, wherein each additional point is no further from any of the first pair of defined points than a first distance defined by the distance indicator.

5. The processing system of claim 4, wherein the step of synthesizing one or more additional points along the first hypothetical line comprises synthesizing at least one additional point to lie outside a portion of the first hypothetical line that spans between the first pair of defined points.

6. The processing system of any of claims 1 to 5, wherein the data processor is configured to synthesize one or more additional points by performing steps comprising:

defining a second hypothetical line, in the Euclidean space system, that passes through at least a second pair of defined points, for the position of the interventional device, in the Euclidean space system; and
synthesizing one or more additional points at one or more locations, in the Euclidean space system, that are no further from a portion of the second hypothetical line that spans between the second pair of defined points than a second distance that is defined by the distance indicator.

7. The processing system of claim 6, wherein the step of synthesizing one or more additional points at one or more locations comprises synthesizing at least one additional point to lie outside the portion of the second hypothetical line that spans between the second pair of defined points.

8. The processing system of any of claims 1 to 7, wherein the data processor is configured to synthesize one or more additional points by performing steps comprising: defining a zone within the Euclidean space system, the position of the zone being defined by the defined points in the Euclidean space system for the position of the interventional device and the size of the zone being defined by the distance indicator; and synthesizing one or more additional points to lie within the defined zone.

9. The processing system of claim 8, wherein the zone has a predetermined shape.

10. The processing system of claim 8, wherein the predetermined shape is based on an expected shape of an electric field generated between the two or more electrodes of the interventional device.

11. The processing system of any of claims 1 to 10, wherein the data processor is configured to define, for each electrical response, a point in the Euclidean space system by performing a process comprising using a mapping function to map the obtained electrical response to a point in the Euclidean space system, wherein the mapping function is configured such that that predetermined properties and/or spatial relationships of the electrodes are maintained.

12. The processing system of any of claims 1 to 11, wherein the data processor is configured to generate the anatomical model by processing the defined points and the one or more synthesized additional points to define the position of the bounds of the anatomical cavity within a 3D volume of discrete voxels representing the Euclidean space system.

13. A computer-implemented method for generating an anatomical model of an anatomical cavity into which an interventional device, carrying two or more electrodes, is positioned, the computer-implemented method comprising:

for each of a plurality of positions of the interventional device:

obtaining an electrical response of each electrode carried by the interventional device to electric fields induced within the anatomical cavity;
defining, for each obtained electrical response, a point in a Euclidean space system based on the electrical response;
obtaining at least one measure of an electrical property, each measure being a measure of the electrical property between a respective pair of electrodes carried by the interventional device or an electrode carried by the interventional device and a reference electrode;
determining, using the at least one measure of the electrical property, a distance between the interventional device and the bounds of the anatomical cavity for each of the plurality of positions; and
synthesizing one or more additional points in the Euclidean space system by processing the defined points in the Euclidean space system and the determined distance between the interventional device and the bounds of the anatomical cavity, and

generating an anatomical model, of the anatomical cavity, by processing the defined points and the one or more synthesized additional points, and
optionally, outputting the anatomical model or a part of the anatomical model.

14. A computer program product comprising code which, when executed by a processor circuit, causes the processor circuit to perform the steps of the method according to claim 13.

15. A non-transitory computer-readable medium or data carrier comprising or carrying the computer program product of claim 14.

FIG. 1

FIG. 2

FIG. 3

FIG. 4

FIG. 5

FIG. 6

FIG. 7

FIG. 8

# EP 4 295 763 A1

EUROPEAN SEARCH REPORT

**Application Number**

EP 22 19 3474

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/138404 A1 (CARBONERA CARLOS [US] ET AL) 30 May 2013 (2013-05-30) * abstract; figures 1-17 * * paragraphs [0008] - [0010], [0029] - [0114] * | 1-15 | INV. A61B5/06 A61B5/107 A61B5/367 A61B5/00 A61N1/40 |
| A | EP 3 922 169 A1 (KONINKLIJKE PHILIPS NV [NL]) 15 December 2021 (2021-12-15) * abstract; figures 1-11 * * paragraphs [0076] - [0272] * | 1-15 | A61B34/20 A61N1/18 G06T19/00 |
| A | US 2012/253161 A1 (HARLEV DORON [US] ET AL) 4 October 2012 (2012-10-04) * abstract; figures 1-13 * * paragraphs [0109] - [0171] * | 1-15 | |
| X,P | EP 4 036 864 A1 (KONINKLIJKE PHILIPS NV [NL]) 3 August 2022 (2022-08-03) * abstract; figures 1-8 * * paragraphs [0008] - [0035], [0039] - [0170] * | 1-15 | |

**TECHNICAL FIELDS SEARCHED (IPC)**

A61B
A61N
G06T

The present search report has been drawn up for all claims

| Place of search | Date of completion of the search | Examiner |
|---|---|---|
| The Hague | 8 May 2023 | Carta, Riccardo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 295 763 A1

## ANNEX TO THE EUROPEAN SEARCH REPORT
## ON EUROPEAN PATENT APPLICATION NO.

EP 22 19 3474

08-05-2023

| Patent document cited in search report | | Publication date | Patent family member(s) | | Publication date |
|---|---|---|---|---|---|
| US 2013138404 | A1 | 30-05-2013 | EP | 2764498 A2 | 13-08-2014 |
| | | | US | 2013138404 A1 | 30-05-2013 |
| | | | WO | 2013081655 A2 | 06-06-2013 |
| EP 3922169 | A1 | 15-12-2021 | NONE | | |
| US 2012253161 | A1 | 04-10-2012 | US | 2010286551 A1 | 11-11-2010 |
| | | | US | 2012253161 A1 | 04-10-2012 |
| | | | US | 2014235986 A1 | 21-08-2014 |
| | | | US | 2017086705 A1 | 30-03-2017 |
| EP 4036864 | A1 | 03-08-2022 | EP | 4036864 A1 | 03-08-2022 |
| | | | WO | 2022161919 A1 | 04-08-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3568068 A1
- EP 0775466 A2
- EP 3607879 A1
- WO 2019215721 A1
- US 2012283715 A1

**Non-patent literature cited in the description**

- **BERGER, MATTHEW et al.** A survey of surface reconstruction from point clouds. *Computer Graphics Forum.*, 2017, vol. 26 (1